(19) Europäisches Patentamt
European Patent Office
Office européen des brevets

(11) Publication number: 0 294 009 A1

# (12) EUROPEAN PATENT APPLICATION

(21) Application number: 88300452.5

(51) Int. Cl.[4]: A61K 7/13

(22) Date of filing: 20.01.88

(30) Priority: 02.06.87 US 57284

(43) Date of publication of application: 07.12.88 Bulletin 88/49

(84) Designated Contracting States: AT BE CH DE ES FR GB GR IT LI LU NL SE

(71) Applicant: LEE PHARMACEUTICALS, INC.
1444 Santa Anita Avenue
South El Monte California(US)

(72) Inventor: Mast, Rolf
11434 Roswell Avenue
Chino California 91710(US)
Inventor: Melger, Helga
900 E. Service Avenue
West Covina California(US)

(74) Representative: Myerscough, Philip Boyd et al
J.A.Kemp & Co. 14, South Square Gray's Inn
London, WC1R 5EU(GB)

## (54) Method of coloring hair.

(57) The invention provides a method of darkening hair using a shampoo containing an accelerator in conjunction with a dressing containing a water soluble lead salt such as lead acetate.

EP 0 294 009 A1

Xerox Copy Centre

## "METHOD OF COLORING HAIR"

There is a class of hair colorants, based on lead acetate, which have the effect of gradually darkening the hair upon repeated application. Some typical formulae for these are given below. These types of hair colorants are also often referred to as hair color restorers. They are generally sold in the men's hair color market but can also be used in women's market.

Although lead acetate products of the current state of the art are supposed to gradually darken the hair, and in fact do so, research has shown that the effect is much too gradual for the majority of users. In many cases, after two weeks of daily product use there is still no perceptible darkening of the hair. This causes much dissatisfaction and attrition from this category.

Although not essential to the art or to this invention technically, it might be mentioned here that the upper amount of lead acetate allowable in these products in the United States of America is set by regulation in the Federal Register/vol. 45, No. 213) Friday, October 13, 1980. Quoting from this regulation:

> "The amount of lead acetate in the cosmetic shall be such that the lead content calculated as Pb, shall not be in excess of 0.6 percent (weight to volume)."

In effect this means that a maximum of 1% lead acetate trihydrate can be used.

There are two types of lead acetate based hair color restorers generally available. The first type are liquid mixtures, the second type, emulsion based cream and lotions. Lead acetate formulation details are not essential at all to this disclosure but typical products will of course be described. In its simplest form the liquid is an aqueous solution of lead acetate, preferably, but not necessarily containing at least some sulfur. If sulfur is present, its concentration is about 0.05 to 10% by weight of the shampoo. Materials commonly incorporated therein are alcohol to speed drying, humectants (such as glycerin and propylene glycol) for hair grooming, fragrances and nonionic surfactants. The latter help with fragrance dissolution as well as adsorption of the product onto the hair. A myriad of formulations and other ingredients are possible including formulation into emulsion based products. These latter are essentially hair creams into which the lead acetate and sulfur have been incorporated.

An earlier reference describing (in the essential details except for the new lead concentration regulations) the currently available products is H. Stanley Redgrove, The Perfumery and Essential Oil Record, June 1940, page 199. A liquid formula given in this reference is as follows:

Lead acetate 1.75 parts by wt.
Milk of sulfur 3.5 parts by wt.
Spirit of Rosemary 2.5 parts by vol.
Glycerin 12.5 parts by vol.
Rosewater 100 parts by vol.

This same reference discusses the formulation of lead acetate based hair creams.

There has been little or no change over the years as evidenced by a more recent patent disclosure (G. Zikuda, DE 2617162, March 9, 1978) which discloses the use of the following liquid formula:

Lead acetate 25 parts
Sulfur 3 parts
Ammonium Chloride 3 parts
Alcohol 3 parts
Glycerin and water to 200 parts

One specific example of a lead acetate cream hair colorant emulsion appears in Micheal & Irene Ash, Formulary of Cosmetic Preparations, Chemical Publishing Co., NY, NY 1977, page 148:

| Material | % by Weight |
|---|---|
| Atmul 84S[1] | 6.5 |
| Dimethylaminopropyl-Palmitamide | 4.5 |
| Tegone A[2] | 0.4 |
| Stearyl alcohol | 3.0 |
| Brij 35[3] | 0.5 |
| Propylene glycol | 5.0 |
| Acetic acid | 0.75 |
| Water | 77.35 |
| Sulfur | 1.0 |
| Lead acetate | 1.0 |

1 glyceryl stearate
2 emollient ester (Goldschmidt Chem.)
3 nonionic surfactant (ICI America)

Essentially this is a hair color restorer - hair dressing combination. The number of possible formula variations which can be made by those skilled in the current art are virtually unlimited. Some idea of these possible variations in emulsifiers, emollients, humectants, perfumes, preservatives, antioxidants, etc. none of which affect the invention herein disclosed, can be obtained by examination of the literature pertaining to hair dressings per se. See for example the above Ash reference pages 134-144, and M. S. Balsam & E. Sagarin, Cosmetics Science and Technology, 2nd. ed., vol. 2, 1972, pages 117-166.

As already stated, the problem with these formulations is that they work much too slowly for consumer satisfaction. This problem has been recognized for many years, but examination of the currently marketed products makes it obvious that a satisfactory solution has not been found. Thus, they are virtually unchanged from formulations given in 1940 and before. Attempts - obviously unsatisfactory - to solve this issue have been referred to in the literature.

The most significant of these attempts, for present purposes, is a two part product where the two parts are simultaneously applied to the hair. Part A is a lead acetate solution and Part B is a solution of ammonium thioglycolate which serves as an accelerator (see Balsam and Sagarin, above, p. 326). This process is reminiscent of women's permanent hair coloring methods where a paraphenylene diamine color precursor, and an accelerator are mixed on the hair to produce an instant color. Neither of these systems are suitable for men's hair colorants for two reasons. Firstly, men need a gradual effect so as to disguise the fact that they are coloring their hair. Secondly, a product which is complicated to use, i.e. where components have to be mixed on the hair, is not well received in the man's market. In addition, thioglycolate solutions are unstable, damage the hair, and develop a high odor which further detracts from their use.

In order to avoid the mixing problem other attempts have been made to incorporate accelerators directly to the lead acetate solution. An example of this is the addition of sodium thiosulfate directly to the aqueous lead acetate. This approach has been mentioned in the H. Stanely Redgrove paper (above), and has surfaced in the recent patent literature, KK Hoyu, Japanese Patent 61148112 July 5, 1986.

In spite of the great need to make a more rapidly acting but not immediate men's hair colorant, these and similar attempts have been pursued over the years and still no suitable formulation is on the market place. As far as we are aware the attempts to incorporate an accelerator directly into the lead acetate solution have resulted in products of unusable chemistry, or products that are no more effective than without the accelerator.

## SUMMARY OF THE INVENTION

Briefly, the present invention comprehends a method of coloring hair which comprises of treating the hair with a conventional water soluble lead salt based hair dressing, and separately with an otherwise conventional shampoo that contains an effective amount of an accelerator containing an oxidizable mercaptan moiety to yield more rapid hair coloring.

It is an object of this invention to provide a new and improved method of coloring hair.

More particularly, it is an object of our invention to provide a new hair coloring method for men which is more rapidly acting.

It is a further object of our invention to provide a new and improved hair coloring method which is simple to follow and which yields results which are more nearly fulfill the wishes of male users of such products.

These and other objects and advantages of our invention will be apparent from the detailed description which follows.

## DESCRIPTION OF PREFERRED EMBODIMENTS

The surprising discovery has been made that by putting certain accelerators in a shampoo, and thus by applying them during a separate shampooing process, exactly the correct speed of color development can be obtained. This invention is all the more surprising, and by the same token all the more useful, when it is considered that (within herein defined limits) the water soluble lead salt composition can be applied at any time, and the hair can then be subsequently shampooed at any other time. This has three very important consequences.

1. The user does not need to follow an unusual regimen in treating their hair.

2. There is no premixing, or any other skill or special instruction necessary in learning how to use the products.

3. Most surprisingly a heretofore unachieved ideal rate of color development is made possible. In particular a color which is approaching the required depth after about two weeks of use.

The essential two items in this hair colorant system are:

1. A conventional water soluble lead salt based hair colorant as described above, and

2. A shampoo which is used during the treatment period, but which is used quite separately from the water soluble lead salt hair dye. A general description of this shampoo composition is now given.

There is no special technology in the shampoo base itself. Anyone skilled in the art can formulate an appropriate shampoo within the other constraints of this invention. As always in matters of this sort an unlimited number of formulations and ingredients are possible. In order to give some practical guidance in these matters, however, the following generalities can be stated. --exclusive of the accelerator which is dealt with separately herein. These descriptions should not be considered as limiting. One essential ingredient is the main foaming detergent. Preferred here are lauryl sulfate and lauryl ether sulfate salts i.e. the sodium, ammonium, and triethanolamine salts. Also commonly used are sodium $C_{14}$-$C_{16}$ olefin sulfonate (an alpha olefin sulfonate), triethanolamine dodecyl benzene sulfonate, sodium lauryl sultaine, sodium methyl cocoyl taurate, $C_{10}$-$C_{14}$ vesions of the above, etc. Other detergents are commonly used as viscosity and foam modifiers. Included in this category are cocamidopropyl betaine, cocamidopropyl hydroxy sultaine, coco betaine, PPG-5-ceteth10 phosphate, cocoamphoacetate, and cocoamphohydroxypropylsulfonate. Another class of very commonly used materials in shampoos are fatty alkanolamides which are used as foam stabilizers and thickening agents. Representative materials in this category are cocamide DEA, cocamide MEA, lauramide DEA, and PEG-6 cocamide. Among the other types of additives that can be incorporated a shampoo are perfumes, sequesterants, acidifiers, opacifiers, hair conditioning agents, antioxidants and microbiological preservatives. A host of other detergents, viscosity modifiers, mildness additives, emollients, hair conditioning agents, foam boosters, colorants, and other additives not related to the essentials of this invention are possible.

In the most general terms the accelerator that goes into the shampoo is any compound containing a readily oxidizable mercaptan radical.

Some examples of mercaptans which function as accelerators are:

$\beta$-aminoethylmercaptan, mercaptoethyl alcohol, metal sulfides, metal hydrosulfides, mercaptoethanesulfonic acid, mercaptobutanesulfonic acid, thioglycolamides, L-thioglycerol, methyl tin mercaptide, butyl tin

mercaptide, dimercaptoadipic acid and 2,4-dithioglutaric acid. These and related molecules are usable in the sense that they contain oxidizable mercapto groups, but they are not equally usable in a practical sense, as some have a very objectionable odor.

One particularly suitable and preferred class of accelerators is based on mercaptan esters in general and especially compounds having the following structure:

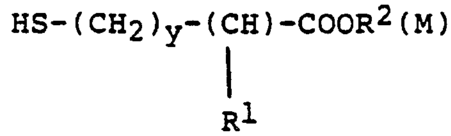

wherein:
y = 1 or 0
$R^1$ = H, $CH_3$, $CH_2COOH(M)$, $NH_2$
$R^2$ = H, $C_1$ to $C_5$ alkyl, glyceryl, $OC_2H_4OH$,$OC_3H_6OH$, or nothing.
When $R^2$ is nothing then:
M = alkali metal ion, an alkaline earth divalent metal ion, an organic amine salt such as guanidine or ethylamine etc., ammonium, or a quaternary ammonium salt.
Note: In monoacidic formulae with an alkaline earth divalent metal ion, the structures are written with the mercapto hydrogen atom missing in order to balance the charge.

Some examples of useful items within this structure are L-cysteine, thiolactic acid, and thiomalic acid. Thioglycolic acid and derivatives are especially preferred.

These accelerators must be incorporated into a shampoo base, but work at a wide effective concentration range of about 0.01% to 10% by weight of the total formula. The preferred range is about 0.02% to 1%.

The following Examples are presented solely to illustrate our invention. In the Examples, parts and percentages are by weight unless a contrary meaning is stated.

## EXAMPLES

All of the following formulations are prepared using conventional and simple techniques. Liquid hair colorant and shampoos by simple mixing, and the cream hair colorants by mixing the oil and water phase ingredients at 75° C with vigorous stirring, and adding perfume sulfur and lead acetate during cooling.

Examples 1 to 5 are representative of typical liquid hair colorant formulations:

All figures are in percent by weight.

| Material | Ex.1 | Ex.2 | Ex.3 | Ex.4 | Ex.5 |
|---|---|---|---|---|---|
| Water | 99.0 | 77.7 | 79.5 | 93.3 | 77.3 |
| SDA-40 alcohol | -- | 10.0 | 8.0 | -- | 10.0 |
| Lead acetate $3H_2O$ | 1.0 | 1.0 | 1.0 | 0.6 | 1.0 |
| Sublimed or precip. sulfur | 1.0 | 1.0 | 0.7 | 0.5 | 1.0 |
| Octoxynol 9 | -- | -- | 0.3 | -- | 0.3 |
| Polysorbate 60 | -- | -- | -- | 0.3 | -- |
| Propylene glycol | -- | 10.0 | 6.0 | 5.0 | -- |
| Glycerin | -- | -- | 4.0 | -- | 10.0 |
| Lactic acid | -- | 0.3 | -- | -- | 0.3 |
| Acetic acid | -- | -- | 0.3 | 0.2 | -- |
| Fragrance | -- | -- | 0.2 | 0.1 | 0.1 |

The above products were prepared by simple mixing at room temperature.

Examples 6 to 8 show three hair color cream formulae that can be used. While a very limited number of examples are given, there are an infinite number of creams that could be prepared with different humectants, emollients, emulsifiers, fragrances, preservatives etc.

| Material | Phase[1] | Ex.6 | Ex.7 | Ex.8 |
|---|---|---|---|---|
| Water | W | 78.65 | 76.2 | 74.3 |
| Lead acetate $3H_2O$ | E | 1.0 | 1.0 | 0.5 |
| Sublimed or precip. sulfur | E | 1.0 | 1.0 | 0.5 |
| Glycerin | W | 8.0 | -- | -- |
| Propylene glycol | W | -- | 5.0 | 7.5 |
| Emerwax 1266[2] | W | 4.0 | -- | -- |
| Ceteth 20 | W | -- | 1.5 | 1.5 |
| Steareth 20 | W | -- | 1.5 | 1.5 |

| Material | Phase[1] | Ex.6 | Ex.7 | Ex.8 |
|---|---|---|---|---|
| Methyl paraben | W | 0.2 | 0.2 | -- |
| Microcrystaline wax | O | 2.0 | -- | -- |
| Mineral oil | O | 1.6 | 5.0 | 6.0 |
| Super Hartolan[3] | O | 0.4 | -- | -- |
| Stearyl alcohol | O | -- | 3.0 | 2.5 |
| Cetyl alcohol | O | 1.5 | -- | -- |
| Glyceryl monostearate | O | -- | 1.5 | 1.5 |
| Propyl parabens | O | 0.05 | 0.1 | -- |
| Octoxynol-9 | W | 0.3 | -- | -- |
| Acetic acid (20% in water) | E | 1.3 | -- | -- |
| Fragrance | E | -- | -- | 0.1 |

1 W = water phase, O = oil phase, E = added to emulsion.
2 Trademark of Emery Ind. Inc.
3 Trademark of Croda Inc.

To prepare the above creams, standard methods are used. The oil phase and the water phase ingredients are separately mixed and heated to about 70 degrees C. With vigorous stirring the oil phase ingredients are then slowly poured into the water phase ingredients. Stirring is continued to about 40 degrees C. Sulfur, fragrance, lead acetate, and acid are added to the emulsion in the 60 to 40 degree C range. It is helpful to add the sulfur as a concentrated slurry with a little humectant (if possible), and the lead acetate as a concentrated aqueous solution with some water taken from the water phase mixture.

With the exception of the accelerators, the shampoos used are generally well known and well used in the art. In the examples below the shampoos are all prepared by simple mixing techniques.

Examples 9 to 14 illustrate shampoos of this invention containing different active materials.

| Material[1] | Ex.9 | Ex.10 | Ex.11 | Ex.12 | Ex.13 | Ex.14 |
|---|---|---|---|---|---|---|
| Sodium laureth sulfate | 18.00 | 18.00 | 18.0 | 18.0 | 18.0 | 18.0 |
| Sodium lauryl sulfate | 10.0 | 10.0 | 10.0 | 10.0 | 10.0 | 10.0 |
| Cocamidopropyl-hydroxysultaine | 8.0 | 8.0 | 8.0 | 8.0 | 8.0 | 8.0 |
| Cocamide DEA | 3.0 | 3.0 | 3.0 | 3.0 | 3.0 | 3.0 |
| Glycerine | 5.0 | 5.0 | 5.0 | 5.0 | 5.0 | 5.0 |
| Lactic acid | 0.6 | 0.6 | 0.6 | 0.6 | 0.6 | 0.6 |
| Water | 54.2 | 54.2 | 54.2 | 54.2 | 54.2 | 54.2 |
| Calcium Thioglycolate | 0.2 | -- | -- | -- | -- | -- |
| Sodium thiolactate | -- | 0.2 | -- | -- | -- | -- |
| Thioglycolic acid | -- | -- | 0.2 | -- | -- | -- |
| L-cysteine | -- | -- | -- | 0.2 | -- | -- |
| Sodium hydrosulfide | -- | -- | -- | -- | 0.2 | -- |
| Sodium sulfide | -- | -- | -- | -- | -- | 0.2 |

1 Fragrance and microbiological preservatives are also preferred ingredients.

Examples 15 to 17 list other shampoo formulations that could be used. The pH of these, as with examples 9 to 14 is in the 4 to 5 range.

Example 15

| Material | % by weight |
|---|---|
| Ammonium lauryl sulfate | 17.0 |
| Cocamidiopropyl hydroxysultaine | 7.4 |
| PPG-5-ceteth-10 phosphate | 4.7 |
| Calcium thioglycolate | 0.3 |
| Lactic acid, (88%) | 0.3 |
| Water | 70.3 |

Example 16

| Material | % by weight |
|---|---|
| Sodium $C_{14-16}$ olefin sulfonate | 25.0 |
| Amphoteric 2 | 7.0 |
| Cocamide DEA | 3.3 |
| Oleic acid | 0.5 |
| Calcium thioglycolate | 0.2 |
| Acetic acid, glacial | 0.2 |
| Water | 63.8 |

Example 17

| Material | % by weight |
|---|---|
| Sodium lauryl ether sulfate | 30.0 |
| Cetyl betaine | 7.0 |
| Coconut fatty diethanolamide | 2.0 |
| Calcium thioglycolate | 0.2 |
| Acetic acid, glacial | 0.2 |
| Water | 60.6 |

Examples 18 to 21 are shampoos adjusted to pH 8 to 9.

Example 18

| Material | % by weight |
|---|---|
| Sodium $C_{14-16}$ olefin sulfonate | 25.0 |
| Amphoteric 2 | 22.0 |
| Polysorbate 20 | 8.0 |
| Laureth 23 | 2.0 |
| Ammonium chloride | 2.0 |
| Fragrance | 0.5 |
| Kathon CG[1] | 0.5 |
| Methyl tin mercaptide | 0.5 |
| Water | 49.5 |

1 Trademark Rohm and Haas

Example 19

| Material | % by weight |
|---|---|
| Sodium lauryl ether sulfate | 50.0 |
| Lauric isopropanolamide | 1.5 |
| Sodium magnesium silicate | 2.0 |
| Fragrance | 0.5 |
| Kathon CG | 0.5 |
| Butyl tin mercaptate | 0.5 |
| Water | 45.0 |

Example 20

| Material | % by weight |
|---|---|
| Sodium methyl cocoyl taurate | 15.0 |
| Cocamide DEA | 6.0 |
| PEG distearate | 1.0 |
| Thioglycolethylamide | 0.2 |
| Water | 77.8 |

Example 21

| Material | % by weight |
|---|---|
| Lauramido propyl betaine | 25.0 |
| Amphoteric 2 | 11.0 |
| Alpha olefin sulfonate | 10.2 |
| Propylene glycol | 0.8 |
| Fragrance | 0.5 |
| Kathon CG | 0.5 |
| Thioglycolethylamide | 0.3 |
| Water | 51.7 |

It should be noted that with all the shampoo formulations above, the accelerator could be added initially to the shampoo or added at a later date, but prior to actual usage. Certainly it is preferred that the accelerator be added initially and in order to preserve the maximum shelf-life full, comparatively oxygen-impermeable packages work best. Thioglycolates and most other of the mercaptan materials are more stable at lower pH's, consequently most of the shampoos are formulated in the pH 4 to 6 range. Although this is not critical to the invention itself it does help practical exploitation to consider the product shelf life. It should be understood therefore that although various salts may be claimed as they are the form of the accelerator that is added to the composition, the ionic species will change depending upon further adjustments made to the shampoo. In particular, the lower the pH, the more the acidic form of any carboxylic acid species will become prevalent.

In order to demonstrate the effectiveness of the subject compositions in vivo, three men of varying degrees of grayness treated their hair daily with compositions of Example 3 or Example 8. A treatment consisted of wetting the hair with the product and then combing the hair, and then allowing the hair to dry. Further, they were instructed to wash the hair either daily, or once every two days with the shampoo of Example 9. In a period of between one and two weeks of this treatment the hair had turned much darker, and the gray hairs had substantially disappeared. The current art uses similar lead salt treatments without the accelerator shampoo. In those circumstances it takes three to four weeks before any substantial coloring of the hair can be observed.

In vitro experiments were also done where hair swatches were treated with different lead salt treatments and washed with different shampoos, with and without the accelerator ingredients. Using a variety of formulations, substantial equivalence was found with the in-vivo experiments described above.

## Claims

1. A method of coloring hair which comprises treating the hair with a water soluble lead salt-based hair dressing, and separately with a shampoo that contains an accelorator having an oxidizable mercaptan moiety.

2. A method according to claim 1 wherein the hair dressing contains 0.05 to 5.0% by weight of the lead salt based on lead, and the shampoo contains 0.01 to 10% by weight of accelerator based on total weight of the shampoo.

3. A method according to claim 1 or 2 wherein the hair dressing also contains up to 10% by weight of fine particulate sulfur.

4. A method according to claim 1, 2 or 3 wherein the accelerator comprises at least one of $\beta$-aminoethylmercaptan, mercaptoethyl alcohol, metal sulfides, metal hydrosulfides, 2,4-dithioglutaric acid, mercaptoethanesulfonic acid, mercaptobutanesulfonic acid, thioglycolamides, L-thioglycerol, methyl tin mercaptide, butyl tin mercaptide and dimercaptoadipic acid.

5. A method according to claim 1, 2 or 3 wherein the accelerator is of the general formula:

$$HS-(CH_2)_y-\underset{\displaystyle R^1}{\underset{|}{(CH)}}-COOR^2$$

wherein y is 1 or 0, $R^1$ is H, $-CH_3$, $-CH_2COOH-CHCOOM$ or $-NH_2$, $R^2$ is H, $C_1$ to $C_5$ alkyl, glyceryl, $OC_2H_4OH$, $OC_3H_6OH$ or M, and M represents where $M^{n+}$ is a cation of valence n selected from alkali metal, alkaline earth metal, organic amine, ammonium and quaternary ammonium ions.

6. A method according to claim 5 wherein the accelerator is thioglycolic acid or an alkali metal or alkaline earth metal salt thereof.

7. A method according to any one of the preceding claims wherein the lead salt is lead acetate.

8. A method according to any one of the preceding claims wherein the hair dressing contains 0.2 to 0.6% by weight of the lead salt based on lead and the shampoo contains 0.02 to 3.0% by weight of accelerator.

European Patent Office

# EUROPEAN SEARCH REPORT

Application Number

EP 88 30 0452

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,D | CHEMICAL ABSTRACTS, vol. 105, no. 18, 3rd November 1986, page 360, no. 158614z, Columbus, Ohio, US; & JP-A-61 148 112 (HOYU CO., LTD) 05-07-1986<br>* Abstract * | 1-8 | A 61 K 7/13 |
| A | FR-A-1 363 266 (REVLON INC.)<br>* Claims; column 4, lines 50-58 * | 1-8 | |
| A | FR-A-1 314 743 (K.K. HOYU SHOKAI)<br>* Claims * | 1-8 | |
| A | CHEMICAL ABSTRACTS, vol. 78, no. 14, 9th April 1973, page 283, no. 88520p, Columbus, Ohio, US; & JP-A-71 31 240 (S. TODA) 10-09-1971<br>* Abstract * | 1-8 | |
| A | BE-A- 477 665 (I. MAGUOLO) | 1-8 | |
| A | FR-A-2 402 445 (SOC. VILLAME-VINCENT)<br>* Page 1, lines 12-19 * | 1-8 | TECHNICAL FIELDS SEARCHED (Int. Cl.4)<br>A 61 K 7/00 |

The present search report has been drawn up for all claims

| Place of search | Date of completion of the search | Examiner |
|---|---|---|
| THE HAGUE | 02-09-1988 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)